# EUROPEAN PATENT SPECIFICATION

(11) **EP 1 372 754 B1**
(45) Date of publication and mention of the grant of the patent: **13.06.2007**
(21) Application number: 02728622.8
(22) Date of filing: 29.03.2002
(51) Int. Cl.: A61L 31/18, A61L 31/02, C22C 38/40

(54) **RADIOPAQUE ALLOY STENT**
TENT AUS EINER RÖNTGENOPAKESLEGIERUNG
STENT EN ALLIAGE RADIO-OPAQUE

(30) Priority: 30.03.2001 US 823308; 15.03.2002 US 364985 P; 28.03.2002 US 112390 P
(43) Date of publication of application: 02.01.2004
(73) Proprietor: Boston Scientific Limited, St. Michael, Barbados, West Indies (BB)
(72) Inventor: CRAIG, Charles, H., Lakeside, CA 92040 (US); TROZERA, Thomas, A., Del Mar, CA 92014 (US); RADISCH, Herbert R., Jr., San Diego, CA 92128 (US)
(74) Representative: Altenburg, Udo
(86) International application number: PCT/US2002/009897
(87) International publication number: WO 2002/078763

(56) References cited:
- EP-A- 0 555 033
- EP-A- 0 604 062
- WO-A-99/39765
- FR-A- 2 500 010
- GB-A- 2 009 236
- US-A- 5 858 556

## Description

### Background of the Invention

Cardiovascular disease is commonly accepted as being one of the most serious health risks facing our society today. Diseased and obstructed coronary arteries can restrict the flow of blood and cause tissue ischemia and necrosis. While the exact etiology of sclerotic cardiovascular disease is still in question, the treatment of narrowed coronary arteries is more defined. Surgical construction of coronary artery bypass grafts (CABG) is often the method of choice when there are several diseased segments in one or multiple arteries. Conventional open-heart surgery is, of course, very invasive and traumatic for patients undergoing such treatment. In many cases, less traumatic, alternative methods are available for treating cardiovascular disease percutaneously. These alternative treatment methods generally employ various types of balloons (angioplasty) or excising devices (atherectomy) to remodel or debulk diseased vessel segments. A further alternative treatment method involves percutaneous, intraluminal installation of one or more expandable, tubular stents or prostheses in sclerotic lesions. Intraluminal endovascular prosthetic grafting is an alternative to conventional vascular surgery. Intraluminal endovascular grafting involves the percutaneous insertion of a tubular prosthetic graft into a blood vessel, and its delivery via a catheter to the desired location within the vascular system. Advantages of the percutaneous revascularization method over conventional vascular surgery include obviating the need for surgically exposing, removing, replacing, or by-passing the defective blood vessel, including heart-lung by-pass, opening the chest, and general anesthesia.

Stents or prostheses are known in the art as implants which function to maintain patency of a body lumen in humans and especially to such implants for use in blood vessels. They are typically formed from a cylindrical metal mesh which expands when internal pressure is applied. Alternatively, they can be formed of wire wrapped into a cylindrical shape. The present application describes an improved stent design which, by its specifically configured struts, can facilitate the deployment and embedment of the stent within a vessel and is constructed from a manufacturing process which provides a controlled and superior stress yield point and ultimate tensile characteristics.

Stents or prostheses can be used in a variety of tubular structures in the body including, but not limited to, arteries and veins, ureters, common bile ducts, and the like. Stents are used to expand a vascular lumen or to maintain its patency after angioplasty or atherectomy procedures, overlie an aortic dissecting aneurysm, tack dissections to the vessel wall, eliminate the risk of occlusion caused by flaps resulting from the intimal tears associated with primary interventional procedure, or prevent elastic recoil of the vessel.

Stents may be utilized after atherectomy, which excises plaque, cutting balloon angioplasty, which scores the arterial wall prior to dilatation, or standard balloon angioplasty to maintain acute and long-term patency of the vessel.

Stents may be utilized in by-pass grafts as well, to maintain vessel patency. Stents can also be used to reinforce collapsing structures in the respiratory, biliary, urological, and other tracts.

Further details of prior art stents can be found in U.S. Patent No. 3,868,956 (Alfidi et. al.); U.S. Patent No. 4,739,762 (Palmaz); U.S. Patent No. 4,512,338 (Balko et. al.); U.S. Patent No. 4,553,545 (Maass et. al.); U.S. Patent No. 4,733,665 (Palmaz); U.S. Patent No. 4,762,128 (Rosenbluth); U.S. Patent No. 4,800,882 (Gianturco); U.S. Patent No. 4,856,516 (Hillstead); U.S. Patent No. 4,886,062 (Wiktor); U.S. Patent No. 5,102,417 (Palmaz); U.S. Patent No. 5,104,404 (Wolff); U.S. Patent No. 5,192,307 (Wall); U.S. Patent No. 5,195,984 (Schatz); U.S. Patent No. 5,282,823 (Schwartz et. al.); U.S. Patent No. 5,354,308 (Simon et. al.); U.S. Patent No. 5,395,390 (Simon et. al), U.S. Patent No. 5,421,955 (Lau et. al.); U.S. Patent No. 5,443,496 (Schwartz et. al.); U.S. Patent No. 5,449,373 (Pinchasik et. al.); U.S. Patent No. 5,102,417 (Palmaz); U.S. Patent No. 5,514,154 (Lau et. al); and U.S. Patent No. 5,591,226 (Trerotola et. al.).

US-A-5858556 discloses a stent made from stainless steel having a radiopaque layer from for example Ta, An or Pt.

WO-A-02/05863 discloses a stent formed from a binary alloy whose primary constituent elements are either tantalum-tungsten or tantalum-niobium.

In general, it is an object of the present invention to provide a stent or prosthesis which can be readily delivered to, expanded and embedded into an obstruction or vessel wall with radiopaque characteristics for fluoroscopic observations during all phases of the interventional procedure.

It is another object of the present invention to provide a stent that is fabricated from alloys that provides better radiopacity than is provided by the known austenitic stainless steels, yet maintains preferred physical properties of austenitic stainless steels.

### Summary of the Invention

The present invention is directed to a radiopaque stent which is relatively flexible along its longitudinal axis to facilitate delivery through tortuous body lumens, but which is stiff enough and stable enough radially in an expanded condition to maintain the patency of a body lumen such as an artery when implanted therein.

The present invention generally relates to virtually any stent design that is manufactured from stainless steel or other materials not having inherent radiopacity properties but which requires increased radiopacity characteristics. For the purposes of this disclosure, the terms "radiopacity" or "radiopaque" refer to a characteristic or material that is opaque to X-ray radiation that renders the material visible under fluoroscopy. Stents are generally delivered and deployed using standard angioplasty techniques (such as employing an over-the-wire or rapid exchange delivery balloon) within the coronary vasculature of the human subject. In this clinical setting, the interventionalist uses angiographic and fluoroscopic techniques that employ X-rays and materials that are radiopaque to the X-rays to visualize the location or placement of the particular device within the human vasculature. Typically, stents are fabricated from a variety of stainless steels, with the 316 series representing a large percentage of the stainless steel used to fabricate currently marketed stents. The typical composition of 316L stainless steel is shown in Table I.

**TABLE I**

| | Component (%) | | | | | | | | |
|---|---|---|---|---|---|---|---|---|---|
| | C | Mn | Si | P | S | Cr | Mo | Ni | Fe |
| Standard 316L | 0.020 | 1.760 | 0.470 | 0.014 | 0.002 | 17.490 | 2.790 | 14.680 | 62.774 |

While the austenitic 300 series stainless steel used for fabricating stents has several beneficial characteristics, such as strength, flexibility, fatigue resistance, biocompatibility, etc., rendering it a good material to make an intravascular stent, one significant disadvantage of 316 series stainless steel, as well as other 300 series of stainless steel, is that they have relatively low radiopaque qualities and, therefore, not readably visual under fluoroscopic observation. It is desirable to utilize a material such as a 300 series stainless steel because of its physical characteristics in fabricating a stent. Yet, the struts of the stent must be relatively thin and, therefore, are poorly visualized under the X-ray fluoroscope. The present invention includes alloys which are a modified stainless steel composition which has improved radiopaque properties while at the same time maintains those characteristics which render stainless steel useful as a material of choice for fabricating stents.

In order to increase the radiopaque characteristics of series 300 stainless steel in the thin sections required to fabricate an intravascular stent, an alloy containing varying amounts of elements that have dense mass and radiopaque characteristics have incorporated into the series 300 chemical structure. The chemical make-up of standard series 300 stainless steel, using series 316 as an example, along with the possible chemical ranges of various such alloys are shown on the following Table.

**TABLE II**

| | Component (%) | | | | | | | | | |
|---|---|---|---|---|---|---|---|---|---|---|
| | C | Mn | Si | P | S | Cr | Mo | Ni | Fe | x |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | 000 - | 10.000 - | 46.185 - | 0.50 - |
| | max. | max. | max. | max. | max. | 20.000 | 3.000 | 18.000 | 74.000 | 10.000 |

| | | | | | | | | | | |
|---|---|---|---|---|---|---|---|---|---|---|
| variable "X" could be Au, Os, Re, Ta, W, Ru or Ir. | | | | | | | | | | |

Although variable X for the various elemental additions is listed in an overall range of 0.5 weight percent to 10 weight percent, the ranges of the individual elements, if selected in the alloy, fall within a specific range for that element different from the range than that listed in the Table, as defined in claims 1, 2 and 4 to 8. It has been found that each individual elemental addition has a different effect with respect to radiopacity and material properties. Thus, each is not equivalent to the other in their function as a radiopacifying agent and contributor to physical properties of a finished stent. In an embodiment as defined in claim 1, gold is added in a range from 2.5 weight percent to 10 weight percent. In a further embodiment as defined in claim 2 which includes osmium as the additional element the range is from 0.5 weight percent to 5 weight percent osmium. If rhenium is selected as the additional element as defined in claim 5, its concentration ranges from 1 weight percent to 5 weight percent. In embodiments incorporating tantalum as defined in claim 6, the concentration of tantalum ranges from 1 weight percent to 2 weight percent. In embodiments incorporating tungsten as defined in claim 7, the concentration of tungsten ranges from 1 weight percent to 4 weight percent. If ruthenium or iridium are selected as defined in claims 8 and 4, the concentration ranges from 2.5 or 2.0 weight precent to 10 weight percent.

Furthermore, palladium may be the added element, as defined in claim 3, and if so its concentration ranges from 2.5 weight percent to 64 weight percent.

The stent, embodying features of the present invention, can be readily delivered to the desired lumenal location by mounting it on an expandable member of a delivery catheter, for example, a balloon or mechanical dilatation device, and passing the catheter/stent assembly through the body lumen to the site of deployment.

Other features and advantages of the present invention will become more apparent from the following detailed description of the invention, when taken in conjunction with the accompanying exemplary drawings.

### Brief Description of the Drawings

Figure 1 is a schematic view of the present invention in its intended operational environment;
Figures 2-8 are illustrations of various designs of prior art surgical stents that could be used in conjunction with the radiopaque alloy of the present invention;
Figure 9 is a schematic view of the present invention showing the radiopaque stent and delivery catheter in a proximal position relative to a lesion. Also shown is the corresponding image of the proximally placed radiopaque stent on a typical cine angiogram or fluoroscopic equipment;
Figure 10 is a schematic view of the present invention showing the radiopaque stent and delivery catheter centered within a lesion in a contracted configuration. Also shown is the corresponding image of the contracted radiopaque stent centered within the lesion on a typical cine angiogram or fluoroscopic equipment;
Figure 11 is a schematic view of the present invention showing the radiopaque stent and delivery catheter centered within a lesion in an expanded configuration. Also shown is the corresponding image of the expanded radiopaque stent centered within the lesion on a typical cine angiogram or fluoroscopic equipment; and
Figure 12 is a schematic view of the present invention showing the expanded and deployed radiopaque stent embedded within a lesion. Also shown is the corresponding image of the embedded radiopaque stent deployed within the lesion on a typical cine angiogram or fluoroscopic equipment.

### Detailed Description

A stent according to the present invention is fabricated from an alloy compound based on a 300 series stainless steel in which a portion of the iron or molybdenum component of the 300 series is replaced with one or a combination of several elements containing improved radiopaque properties. These elements are gold (Au), osmium (Os), palladium (Pd), rhenium (Re), tantalum (Ta), tungsten (W), Ruthenium (Ru) or Iridium (Ir). This group consists of elements with dense masses. The dense mass provides these materials with improved absorption of X- rays, thus providing improved radiopaque characteristics. By including one or more of these elements in a modified series 300 stainless steel, X-rays employed in angiogram procedures or cineograms allow the visualization of the stent during all phases of a standard clinical procedure. The alloy for fabricating stents contains a range of 0.5 to 10.0 percent of Au, Os, Re, Ta, W, Ru or Ir varying depending on which element is selected, or 2.5 to 64 percent Pd, as it has been found that each element has a unique effect on radiopacity and physical properties of the stent. Replacing too much of the radiopaque element with the iron or molybdenum component could possibly decrease the beneficial qualities of a 300 series stainless steel for manufacturing stents without contributing significantly to improved radiopaque characteristics. It is anticipated that various combinations of the radiopaque elements can be used to replace the iron or molybdenum component without adversely affecting the ability to form austenite.

The foregoing, as well as additional advantages of the present invention, is achieved by employing various amounts of selected elemental additions to achieve alloyed stents of sufficient radiopacity and maintain adequate structural properties. The formulations of preferred alloys are summarized in Tables III through X below, containing in weight percent:

**TABLE III**

| | Component (%) | | | | | | | | | |
|---|---|---|---|---|---|---|---|---|---|---|
| | C | Mn | Si | P | S | Cr | Mo | Ni | Fe | Au |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | .000 - | 10.000 - | 46.185 - | 2.5 - |
| | max. | max. | max. | max. | max. | 20.000 | 3.000 | 18.000 | 74.000 | 10.0 |

As indicated above, it has been found that embodiments of the alloy of the present invention include gold in a concentration of 2.5 weight percent to 10 weight percent. It has been found that gold can be added within this range without affecting the normal austenite and ferrite phase distributions of the alloy. At concentrations greater than 5 percent, gold rich precipitates were formed in the alloy, but were fine and discontinuous and believed not to have any adverse affect on physical properties. At greater than 10 percent, the precipitates become as large as the austenite grains and would be expected to reduce tensile strength and ductility. The minimum recommended concentration of gold to obtain adequate radiopacity enhancement is 2.5 percent.

**TABLE IV**

| | Component (%) | | | | | | | | | |
|---|---|---|---|---|---|---|---|---|---|---|
| | C | Mn | Si | P | S | Cr | Mo | Ni | Fe | Os |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | .000 - | 10.000 - | 46.185 - | 0.5 - |
| | max. | max. | max. | max. | max. | 20.000 | 3.000 | 18.000 | 74.000 | 5.0 |

As indicated in Table IV above, an embodiment incorporating osmium into the alloy contains a concentration of 0.5 weight percent to 5 weight percent osmium. Osmium is a relatively heavy element compared to others disclosed herein, and it has been found that 0.5 weight percent osmium provides sufficient radiopacity enhancement. It is also a potent hardening element and believed only tolerated in the alloy up to 5 weight percent without reducing the ductility of the final stent to too low of a level.

**TABLE V**

| | Component (%) | | | | | | | | | |
|---|---|---|---|---|---|---|---|---|---|---|
| | C | Mn | Si | P | S | Cr | Mo | Ni | Fe | Pd |
| Standard 316 | 0.020 | 1.760 | 0.470 | 0.014 | 0.002 | 17.490 | 2.790 | 14.680 | 62.774 | 0.000 |
| Modified 316 | 0.030 max. | 2.000 max. | 0.750 max. | 0.023 max. | 0.010max. | 12.000 - 20.000 | .000 - 3.000 | 10.000 - 18.000 | substantial portion of the balance | 2.5 - 64.0 |

As indicated in the above Table, when palladium is utilized as the additional element in the alloy, the concentration can range from 2.5 weight percent to 64.0 weight percent. It is believed that 2.5 weight percent provides sufficient radiopacity enhancement to be advantageous. Further, palladium is soluble in austenitic iron up to 64 weight percent and above this, it forms intermetallic phases that would be deleterious to mechanical properties of a final stent.

**TABLE VI**

| | Component (%) | | | | | | | | | |
|---|---|---|---|---|---|---|---|---|---|---|
| | C | Mn | Si | P | S | Cr | Mo | Ni | Fe | Re |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | .000 - | 10.000 - | 46.185 - | 1.0- |
| | max. | max. | max. | max. | max. | 20.000 | 3.000 | 18.000 | 74.000 | 5.0 |

As indicated in the Table above, when rhenium is selected as the element alloy with stainless steel, its concentration can range from 1 weight percent to 5 weight percent. Rhenium is a ferrite phase stabilizer in iron. At concentrations of 5 weight percent, it is believed to overcome the beneficial effects of nickel as an austenite stabilizer and would result in the formation of significant amounts of ferrite phase. It is believed, however, that an addition of 1 weight percent would provide sufficient increased radiopacity to the stent.

**TABLE VII**

| | Component (%) | | | | | | | | | |
|---|---|---|---|---|---|---|---|---|---|---|
| | C | Mn | Si | P | S | Cr | Mo | Ni | Fe | Ta |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000- | .000- | 10.000 - | 46.185 - | 1.0 - |
| | max. | max. | max. | max. | max. | 20.000 | 3.000 | 18.000 | 74.000 | 2.0 |

As indicated in the Table above, when tantalum is selected as the additional element, its concentration can range from 1 weight percent to 2 weight percent. Tantalum at 1 weight percent is the minimum concentration to obtain the least amount of radiopacity enhancement that would be advantageous. At concentrations above 2 weight percent, a brittle phase forms and fenite stabilization can occur, leading to a reduction in mechanical properties of the stent.

**TABLE VIII**

| | Component (%) | | | | | | | | | |
|---|---|---|---|---|---|---|---|---|---|---|
| | C | Mn | Si | P | S | Cr | Mo | Ni | Fe | W |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | .000 - | 10.000 - | 46.185 - | 1.0- |
| | max. | max. | max. | max. | max. | 20.000 | 3.000 | 18.000 | 74.000 | 4.0 |

As indicated in the above Table, when tungsten is selected as the additional element for the alloy, its concentration can range from 1 weight percent to 4 weight percent. The minimum recommended concentration of 1 weight percent is utilized to obtain the least amount of radiopacity enhancement that would be advantageous. At concentrations above 4 weight percent, brittle phase formation and ferrite stabilization can occur that would lead to a reduction in mechanical properties.

**TABLE IX**

| | Component (%) | | | | | | | | | |
|---|---|---|---|---|---|---|---|---|---|---|
| | C | Mn | Si | P | S | Cr | Mo | Ni | Fe | Ru |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.01 | 12.000- | .000- | 10.000 - | 46.185 - | 2.5 - |
| | max. | max. | max. | max. | max. | 20.000 | 3.000 | 18.000 | 74.000 | 10.0 |

As indicated in the above Table, when ruthenium is selected as the elemental addition to the alloy, its concentration ranges from 2.5 weight percent to 10 weight percent. It is believed that 2.5 weight percent is the minimum recommended concentration to obtain the least amount of radiopacity enhancement that would be advantageous. At concentrations up to 10 weight percent, the ruthenium is completely soluble in the alloy, but above the level, the undesirable sigma phase can form, leading to cracking during formation.

**TABLE X**

| | Component (%) | | | | | | | | | |
|---|---|---|---|---|---|---|---|---|---|---|
| | C | Mn | Si | P | S | Cr | Mo | Ni | Fe | Ir |
| Standard 316 | 0.020 | 1.760 | 0.470 | 0.014 | 0.002 | 17.490 | 2.790 | 14.680 | 62.774 | 0.000 |
| Modified 316 | 0.030 | 2.000 | 0.750 | 0.023 | 0.010 | 12.000 - | .000 - | 10.000 - | 46.185 - | 2.0 - |
| | max. | max. | max. | max. | max. | 20.000 | 3.000 | 18.000 | 74.000 | 10.0 |

As indicated in the above Table, when iridium is selected as the element for addition to the alloy, its concentration can range from 2 weight percent to 10 weight percent. It has been found that 2 weight percent is the minimum requirement to achieve sufficient radiopacity enhancement to be advantageous. At concentrations greater than 10 weight percent, it is believed that the mechanical properties of the final stent would be adversely affected.

The austenitic alloy for fabricating the present stent with improved radiopaque properties can contain up to 0.03% of carbon. High concentrations of the carbon element can create iron carbides which precipitate at the grain boundaries, resulting in reduced mechanical and corrosion properties. Therefore, too much carbon adversely affects the fracture toughness of this alloy.

Chromium contributes to the good hardenability corrosion resistance and hardness capability of this alloy and benefits the desired low ductile-brittle transition temperature of the alloy. Therefore, at least 12% chromium, and preferably at least about 17.5% chromium, is present. Above 20% chromium, the alloy is susceptible to rapid overaging such that the unique combination of high tensile strength and high fracture toughness is not attainable. In addition, an alloy with a high percentage of chromium can result in the leaching of Cr ions.

Nickel contributes to the hardenability of this alloy such that the alloy can be hardened with or without rapid quenching techniques. In this capacity, nickel benefits the fracture toughness and stress corrosion cracking resistance provided by this alloy and contributes to the desired low ductile-to-brittle transition temperature. Furthermore, nickel also is an austenitic stabilizer, thereby encouraging that the face-centered cubic structure is maintained during the cooling process of the alloy. Accordingly, at least 10.0% nickel, and preferably at least about 14.7% nickel, is present. Above 18% nickel, the fracture toughness and impact toughness of the alloy can be adversely affected because the solubility of carbon in the alloy is reduced which may result in carbide precipitation in the grain boundaries when the alloy is cooled at a slow rate, such as when air cooled following forging. In addition, an alloy with a high percentage of nickel can result in the leaching of Ni ions, an element known to be toxic to human and animal tissues. Therefore, using a stainless steel alloy for fabricating stents with a relatively high percent of certain components, such as nickel (Ni) or chromium (Cr), could result in leaching of Ni or Cr ions to human tissues. This leaching of toxins is exacerbated by laser cutting techniques used for fabricating stent design from tubular members.

Molybdenum is present in preferred alloys because it benefits the desired low ductile brittle transition temperature of the alloy. Its primary benefit, however, is to enhance the corrosion resistance of the alloy. In addition, molybdenum is a ferrite stabilizer and can have a deleterious effect on the stabilization of the desired austenitic structure. Above 3% molybdenum the fracture toughness of the alloy is adversely affected. Preferably, molybdenum is limited to not more than about 1.2%. However, either a portion or the entire percent of the molybdenum can be replaced with certain radiopaque elements such as Pt, Au, Os, Pd, or W without adversely affecting the desired characteristics of the alloy.

The austenitic 300 stainless steel alloy for fabricating the present invention stent with radiopaque properties can also contain up to 2.0% manganese. Manganese is an austenitic stabilizer and is partly depended upon to maintain the austenitic, nonmagnetic character of the alloy. Manganese helps assure that the face-centered cubic structure is maintained during cooling process of the alloy. Manganese also plays a role, in part, in providing resistance to corrosive attack.

The balance of the alloy, according to the present invention, is essentially iron except for the usual impurities found in commercial grades of alloys intended for similar service or use. The levels of such elements must be controlled so as not to adversely affect the desired properties of this alloy. The alloy for fabricating a series 300 stainless steel alloy with radiopaque properties can contain up to 0.75% silicon. Furthermore, the alloy for fabricating a series 300 stainless steel stent with radiopaque properties contains up to 0.023% and 0.01% phosphorus and sulfur, respectively, without affecting the desirable properties.

No special techniques are required in melting, casting, or working the alloy for fabricating the present invention stent. Induction heating is the preferred method of melting and refining the alloy used in fabricating the present invention stent. Arc melting followed by argon-oxygen decarburization is another method of melting and refining, but other practices can be used. In addition, this alloy can be made using powder metallurgy techniques, if desired. This alloy is also suitable for continuous casting techniques.

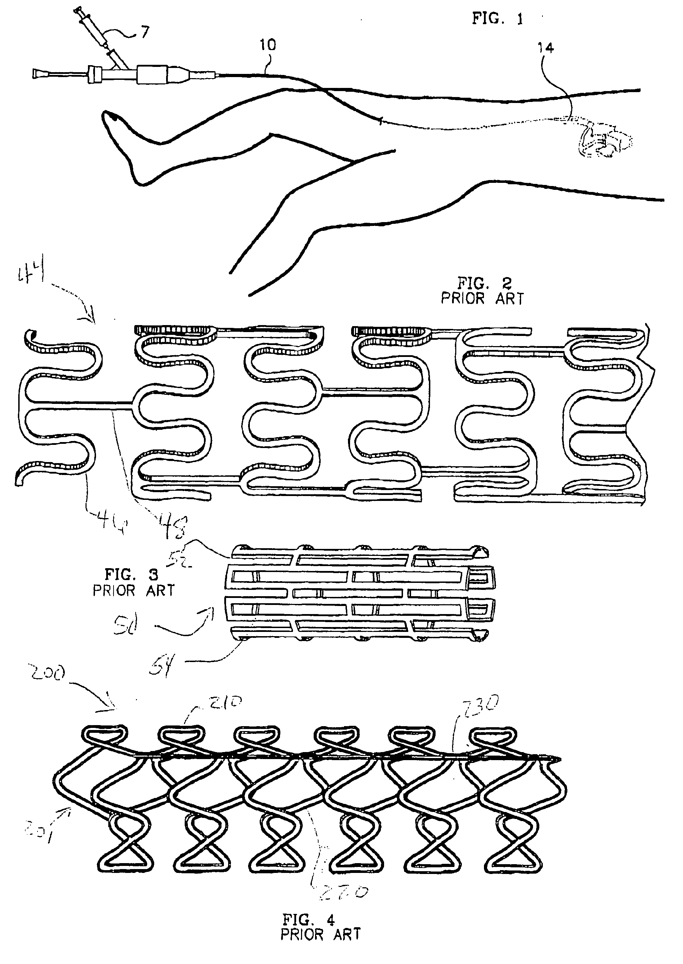

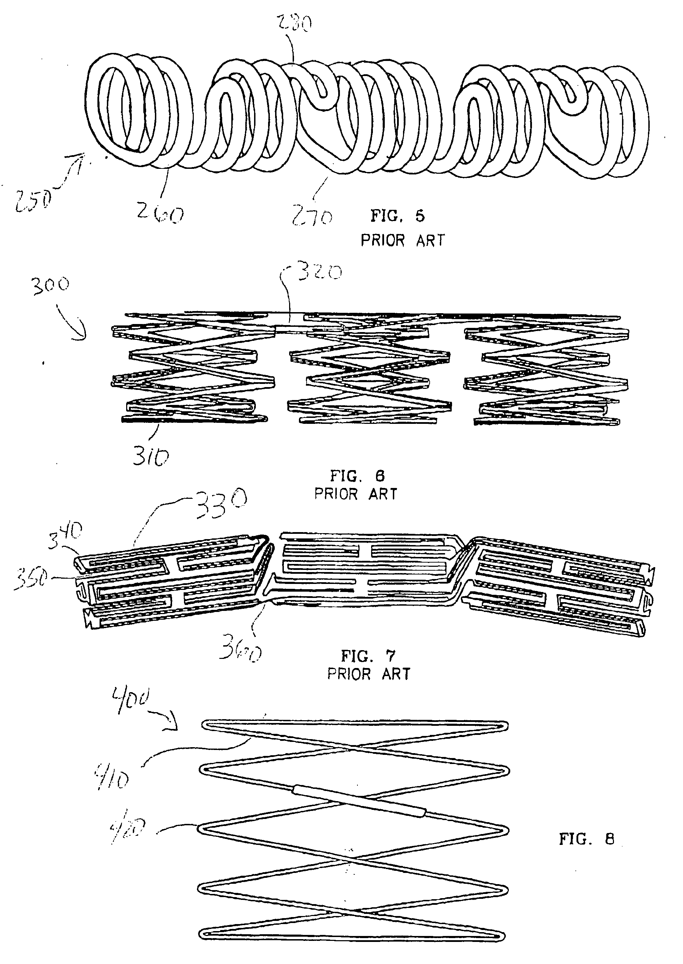

Now referring to Figure 1, presented is an environmental example that is directed to an expandable stent 44 which is relatively flexible along its longitudinal axis to facilitate delivery through tortuous body lumens, but which is stiff and stable enough radially in an expanded condition to maintain the patency of a body lumen, such as an artery when implanted therein. Figure 1 depicts a delivery system 10 for percutaneously implanting the stent in a coronary artery via the femoral artery. An inflation device 7 is depicted for inflating the balloon on a preferred delivery system 10 for stent expansion. As shown in Figure 2, the stent 44 generally includes a plurality of radially expandable loop elements 46 which are relatively independent in their ability to expand and to flex relative to one another. Interconnecting elements or backbone 48 extends between the adjacent loop elements 46 to provide increased stability and a preferable position for each loop to prevent warping of the stent upon the expansion thereof. The resulting stent structure is a series of radially expandable loop elements 46 which are spaced longitudinally close enough so that the obstruction, vessel wall, and any small dissections located at the treatment site of a body lumen may be dilated or pressed back into position against the lumenal wall. The individual loop elements 46 may bend relative to adjacent loop elements 46 without significant deformation, cumulatively providing a stent which is flexible along its length and about its longitudinal axis, but is still very stiff in the radial direction in order to resist collapse.

It should be noted that the stent is expanded from a contracted configuration to achieve an expanded configuration by "deforming" certain elements. By use of the term "deformed" it is meant that the material from which the stent is manufactured is subjected to a force which is greater than the elastic limit of the material utilized to make the expandable elements. Accordingly, the force is sufficient to permanently or semi-pemianently bend the expandable elements wherein the diameter of the stent increases from the first diameter, d, to the expanded diameter, d¹. The force to be applied to the expandable elements must be sufficient to not "spring back" and assume the contracted or partially contracted configuration. Therefore, the expanded stent retains the expanded configuration and is relatively rigid in the sense of having an outer shape maintained by a fixed framework, and not pliant.

The open reticulated structure of the stent allows for a large portion of the vascular wall to be exposed to blood which can improve the healing and repair of any damaged vessel lining. It is desirable that the stent struts be relatively thin in cross-section to minimize overall profile, yet have enough radial and tensile strength to maintain vessel patency after stent deployment. In order to achieve the radiopaque characteristics desired for clinical procedures, other stent designs must compromise some of the preferred characteristics by increasing the cross-sectional thickness of all the struts, increasing the cross-sectional thickness of some of the struts, or employing other materials for fabrication that fail to have the preferred characteristics of series 300 stainless steels. By fabricating a stent design with the unique alloy described herein, one can optimize the physical and mechanical parameters of the stent design for clinical utility without compromising the desired stent characteristics.

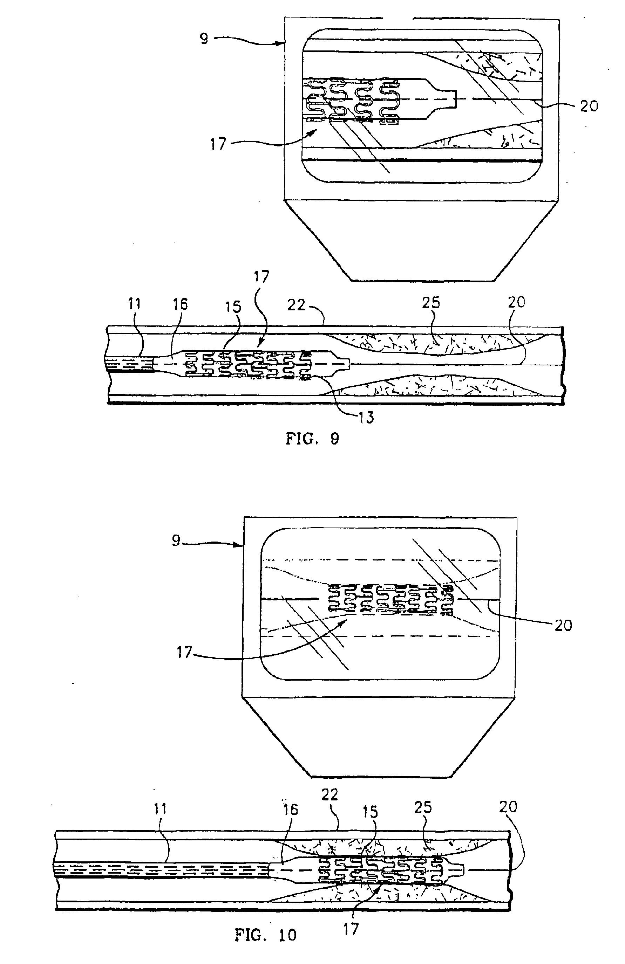

As per the stent design previously described herein, there is a variety of other stent designs. However, the present invention is not limited to any particular design. However, further examples, as shown in Figures 3-8, are given below to further facilitate use of the invention. Figure 3 depicts a stent including struts 52 in a generally rectangular pattern with interstitial spaces therebetween. U.S. Patent Nos. 4,886,062 and 5,133,732 to Wiktor (see Figure 4) describe a stent 200 with a cylindrical body 201 formed of generally continuous wire 210 having a deformable zigzag 220, wherein the wire is a coil of successive windings and the zigzag and is in the form of a sinusoidal wave, wherein the stent body may be expanded from the first unexpended diameter to a second expanded diameter by the force of an inflating balloon. There are also means such as hooks 230 for preventing the stent body from stretching along its longitudinal axis. U.S. Patent No. 4,969,458 to Wiktor (see Figure 5) shows a stent 250 which is a wire 260 winding in a hollow cylindrical shape. The winding includes a series of groups of helical coils 270 along the length of the winding while providing radial strength. The coils of each group are wound in a direction opposite to the winding of the next adjacent group of coils. A reversely turned loop 280 joining each to successive group allows for smooth expansion of the adjacent group of coils. U.S. Patent No. 5,282,823 to Schwartz (see Figure 6) shows a stent 360 comprising a cylindrical-shaped body which comprises a plurality of substantially helical metal elements 310 joined to allow flexing of the stent along its longitudinal axis. The helical wire winding is substantially continuous, and there is a polymeric connector 320 extending between the helical metal elements to provide strain relief means. U.S. Patent No. 5,104,404 to Wolff is similar. U.S. Patent No. 5,102,417 is similar in design to U.S. Patent No. 5,195,984 described earlier hereinabove. U. S. Patent No. 5,102,417 (see Figure 7) shows a plurality of expandable and deformable vascular grafts 330 which are thin wall tubular members 340 having a plurality of slots 350 disposed substantially parallel to the longitudinal axis of the tubular members and adjacent grafts are flexibly connected by at least one connector member 360. U.S. Patent Nos. 5,102,417; 4,739,762; 4,733,665 and 4,776,337 are all by Palmaz. The Palmaz patents are similar in design to the '417 and '984 patents described earlier hereinabove. U.S. Patent No. 4,580,568 to Gianturco (see Figure 8) describes a stent 400 comprising a wire formed into a closed zigzag configuration including an endless series of straight sections 410 and a plurality of bends 420. The straight sections are joined by the bends to form the stent. The stent is resiliently depressible into a small first shape wherein the straight sections are arranged side by side and closely adjacent one another for insertion into a passageway, and the stressed bends are stored therein.

The present stent can be manufactured from a tubular member made from one of the cited stainless steel alloys described herein using various manufacturing techniques. However, the present invention is not limited to any particular fabrication method. The following examples are given below to further facilitate use of the invention. There are several manufacturing techniques which can transform a tubular member into a particular stent design: 1) photo-mask and etch techniques as described in U.S. Patent No. 5,902,475; 2) a laser ablation/etching process disclosed in U.S. Patents Nos. 6,066,167; 6,056,776; 5,766,238; 5,735,893; 5,514,154 or 5,421,955; and 3) utilizing a laser to directly cut away metal and form the pattern into a tubular member. Any one of these manufacturing examples can be used to produce the present invention stent with the radiopaque stainless steel alloy.

In the photo mask and etch process the outer surface of a tubular member is uniformly coated with a photosensitive resist. This coated tubular member is then placed in an apparatus designed to rotate the tubular member while the coated tubular member is exposed to a designated pattern of ultraviolet (UV) light. The UV light activates the photosensitive resist causing the areas where UV light is present to expose (cross-link) the photosensitive resist. The photo-sensitive resist forms crosslinks where is it exposed to the UV light, thus forming a pattern of hardened and cured polymer which mimics the particular stent design surrounded by uncured polymer. The film is adaptable to virtually an unlimited number of intricate stent designs. The process from the apparatus results in the tubular member having a discrete pattern of exposed photosensitive material with the remaining areas having unexposed photosensitive resist.

The exposed tubular member is immersed in a resist developer for a specified period of time. The developer removes the relatively soft, uncured photosensitive resist polymer and leaves behind the cured photosensitive resist which mimics the stent pattern. Thereafter, excess developer is removed from the tubular member by rinsing with an appropriate solvent. At this time, the entire tubular member is incubated for a specified period of time, allowing the remaining photosensitive resist polymer to fully cure and bond to the surface of the processed tubular member.

The processed tubular member is then exposed to an electrochemical etching process which removes uncovered metal from the tubular member, resulting in the final tubular member or stent configuration.

In an example of the laser/etching process, a tubular member is coated with a resist and placed in a rotatable collet fixture of a machine-controlled apparatus for positioning the tubular member relative to a laser. Then, according to the machine coded instructions, the tubing is rotated and moved longitudinally relative to the laser which is also machine controlled wherein the laser selectively removes the resistant coating on the tubular member by ablation. A stent pattern is fonned on the surface of the tubular member that is created by a subsequent chemical etching process.

In an example of the direct laser method, a tubular member is placed in a collet fixture of a machine-controlled apparatus for positioning the tubular member relative to a laser. Then, according to the machine coded instructions, the tubing is rotated and moved longitudinally relative to the laser which is also machine controlled wherein the laser selectively ablates and removes metal forming the stent pattern.

Figure 9 presents a schematic view of the present invention showing the radiopaque stent 17 in a proximal position to the lesion 25. Attached to distal catheter shaft 11 is the delivery balloon with proximal end 16 and distal end 13. Mounted on the delivery balloon is a representation of the present invention stent 17 with unexpended struts 15 in a non-expanded configuration. A previously placed guidewire 20 transects the catheter shaft and extends beyond lesion 25. Also shown is the corresponding image of the proximally placed radiopaque stent 17 on a typical cine angiogram or fluoroscopic equipment 9.

In the next stent of a typical clinical procedure, the stent/deliveiy system is advanced to a position where it becomes centered within the lesion 25, as shown in Figure 10. Also shown is the corresponding image of the contracted radiopaque stent centered within the lesion on a typical cine angiogram or fluoroscopic equipment.

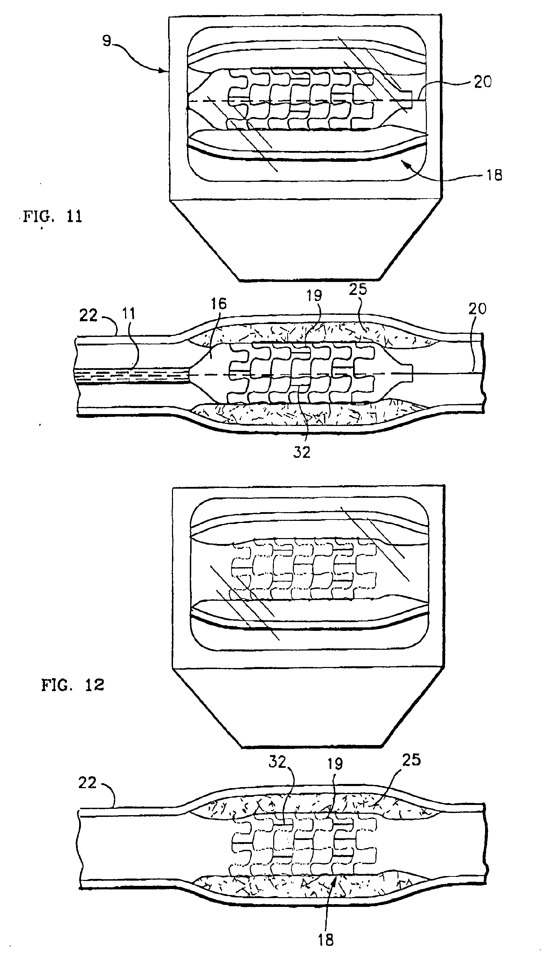

Figure 11 is a schematic view of the present invention showing the radiopaque stent and delivery catheter centered within the lesion in an expanded configuration. Also shown is the corresponding image of the expanded radiopaque stent centered within the lesion on a typical cine angiogram or fluoroscopic equipment.

Figure 12 is a schematic view of the present invention showing the expanded and deployed radiopaque stent embedded within the lesion. Also shown is the corresponding image of the embedded radiopaque stent deployed within the lesion on a typical cine angiogram or fluoroscopic equipment.

It is apparent from the foregoing description and the accompanying examples, that the alloy according to the present invention provides a unique combination of tensile strength and radiopaque characteristics not provided by known series 300 stainless steel alloys. This stent invention is well suited to applications where high strength, biocompatibility and radiopacity are required.

Various modifications are possible within the scope of the invention claimed.

## Claims

1. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
2.5-10.0 Au
with the balance of the alloy being essentially iron except for the usual impurities.

2. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
0.5-5.0 Os
with the balance of the alloy being essentially iron except for the usual impurities.

3. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
2.5-64.0 Pd
with the balance of the alloy being essentially iron except for the usual impurities.

4. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
2.0-10.0 Ir
with the balance of the alloy being essentially iron except for the usual impurities.

5. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
1.0-5.0 Re
with the balance of the alloy being essentially iron except for the usual impurities.

6. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
1.0-2.0 Ta
with the balance of the alloy being essentially iron except for the usual impurities.

7. An intravascular stent manufactured from an alloy which provides increased radiopaque characteristics, said alloy comprising, in weight percent,
0.030 max. C
2.000 max. Mn
0.750 max. Si
0.023 max. P
0.010 max. S
12.000-20.000 Cr
0.000-3.000 Mo
10.000-18.000 Ni
1.0-4.0 W
with the balance of the alloy being essentially iron except for the usual impurities.

8. An intravascular stent manufactured from an alloy which provides increased radiopaque characteristics, said alloy comprising of, in weight percent,
0.030 max. C
2.000 max. Mn
0.750 max. Si
0.023 max. P
0.010 max. S
12.000-20.000 Cr
0.000-3.000 Mo
10.000-18.000 Ni
2.5-10.0 Ru
46.185-74.000 Fe.

## Patentansprüche

1. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
2.5-10.0 Au
wobei das Hauptgewicht der Legierung im Wesentlichen aus Eisen besteht, ausgenommen die üblichen Verunreinigungen.

2. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
0.5-5.0 Os
wobei das Hauptgewicht der Legierung im Wesentlichen aus Eisen besteht, ausgenommen die üblichen Verunreinigungen.

3. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
2.5-64.0 Pd
wobei das Hauptgewicht der Legierung im Wesentlichen aus Eisen besteht, ausgenommen die üblichen Verunreinigungen.

4. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
2.0-10.0 Ir
wobei das Hauptgewicht der Legierung im Wesentlichen aus Eisen besteht, ausgenommen die üblichen Verunreinigungen.

5. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
1.0-5.0 Re
wobei das Hauptgewicht der Legierung im Wesentlichen aus Eisen besteht, ausgenommen die üblichen Verunreinigungen.

6. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
1.0-2.0 Ta
wobei das Hauptgewicht der Legierung im Wesentlichen aus Eisen besteht, ausgenommen die üblichen Verunreinigungen.

7. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
1.0-4.0 W
wobei das Hauptgewicht der Legierung im Wesentlichen aus Eisen besteht, ausgenommen die üblichen Verunreinigungen.

8. Intravaskulärer Stent, hergestellt aus einer Legierung, welche erhöhte strahlenundurchlässige Eigenschaften hat, wobei die Legierung in Gewichtsprozenten aufweist:
0.030 max. C
2.000 max. Mn
0.750 max. Si
0.023 max. P
0.010 max. S
12.000-20.000 Cr
0.000-3.000 Mo
10.000-18.000 Ni
2.5-10.0 Ru
46.185-74.000 Fe.

## Revendications

1. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 pour cent à 20,000 pour cent de Cr
de 0,000 pour cent à 3,000 pour cent de Mo
de 10,000 pour cent à 18,000 pour cent de Ni
de 2,5 pour cent à 10,0 pour cent d'Au
le reste de l'alliage étant essentiellement du fer à l'exception des impuretés habituelles.

2. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 0,5 à 5,0 pour cent d'Os
le reste de l'alliage étant essentiellement du fer à l'exception des impuretés habituelles.

3. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 2,5 à 64,0 pour cent de Pd
le reste de l'alliage étant essentiellement du fer à l'exception des impuretés habituelles.

4. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 2,0 à 10,0 pour cent d'Ir
le reste de l'alliage étant essentiellement du fer à l'exception des impuretés habituelles.

5. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 1,0 à 5,0 pour cent de Re
le reste de l'alliage étant essentiellement du fer à l'exception des impuretés habituelles.

6. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 1,0 à 2,0 pour cent de Ta
le reste de l'alliage étant essentiellement du fer à l'exception des impuretés habituelles.

7. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 1,0 à 4,0 pour cent de W
le reste de l'alliage étant essentiellement du fer à l'exception des impuretés habituelles.

8. Endoprothèse intravasculaire fabriquée à partir d'un alliage qui offre des caractéristiques radio-opaques accrues, ledit alliage comprenant, en pourcentage en poids,
0,030 pour cent de C au maximum
2,000 pour cent de Mn au maximum
0,750 pour cent de Si au maximum
0,023 pour cent de P au maximum
0,010 pour cent de S au maximum
de 12,000 à 20,000 pour cent de Cr
de 0,000 pour cent à 3,000 pour cent de Mo
de 10,000 pour cent à 18,000 pour cent de Ni
de 2,5 à 10,0 pour cent de Ru
de 46,185 pour cent à 74,000 pour cent de Fe.
